# EUROPÄISCHE PATENTSCHRIFT

(11) **EP 2 318 076 B1**
(45) Veröffentlichungstag und Bekanntmachung des Hinweises auf die Patenterteilung: **26.11.2014**
(21) Anmeldenummer: 09777951.6
(22) Anmeldetag: 18.08.2009
(51) Int. Cl.: A61M 5/44

(54) **HEIZUNG EINES MEDIZINISCHEN GERÄTS, MEDIZINISCHES GERÄT MIT EINER HEIZUNG SOWIE VERFAHREN ZUM AUFRÜSTEN EINES MEDIZINISCHEN GERÄTS**
HEATING OF A MEDICAL DEVICE, MEDICAL DEVICE COMPRISING A HEATER AND METHOD FOR UPGRADING A MEDICAL DEVICE
DISPOSITIF DE CHAUFFAGE D'UN APPAREIL MÉDICAL, APPAREIL MÉDICAL ÉQUIPÉ DU DISPOSITIF DE CHAUFFAGE ET PROCÉDÉ POUR ÉQUIPER LEDIT APPAREIL MÉDICAL

(30) Priorität: 27.08.2008 DE 102008039918
(43) Veröffentlichungstag der Anmeldung: 11.05.2011
(73) Patentinhaber: Fresenius Medical Care Deutschland GmbH, 61352 Bad Homburg (DE)
(72) Erfinder: MAI, Erwin, 90491 Nürnberg (DE); OESTERREICH, Stefan, 61267 Neu-Ansprach (DE)
(74) Vertreter: Laufhütte, Dieter
(86) Internationale Anmeldenummer: PCT/EP2009/005985
(87) Internationale Veröffentlichungsnummer: WO 2010/025824

(56) Entgegenhaltungen:
- EP-A1- 1 808 190
- WO-A1-84/04042
- WO-A1-95/20985
- WO-A1-99/26690
- WO-A2-2007/120812
- DE-C1- 19 503 350
- FR-A1- 2 518 884
- FR-A1- 2 791 126
- US-A- 3 475 590
- US-A- 4 574 876
- US-A- 5 125 069
- US-A- 5 616 268
- US-A1- 2003 220 598
- US-A1- 2004 190 885

## Beschreibung

Die vorliegende Erfindung betrifft eine Heizung eines medizinischen Geräts, ein medizinisches Gerät mit einer Heizung, ein Verfahren zum Aufrüsten eines medizinischen Gerätes, ein Schlauchset mit wenigstens einem Heizbeutel sowie ein System umfassend wenigstens eine Heizung, siehe z.B. US-A-5 125 069.

Bei medizinischen Geräten wie beispielsweise Maschinen zur extrakorporalen Blutbehandlung, insbesondere Dialysemaschinen, ist es bereits bekannt, z.B. Bauteile bzw. Einmalbauteile eines flüssigkeitsführenden Systems in eine Heizung einzulegen, um die im Flüssigkeitssystem befindliche Flüssigkeit auf- bzw. anzuheizen.

Dabei ist beispielsweise bei Dialysemaschinen auf einer Bedienseite eine Heizung vorgesehen, in die sogenannte Heizbeutel einzudrücken sind. Die Heizbeutel sind z.B. Bestandteil eines Schlauchsystems eines Dialysatsystems und/oder eines Substituatsystems.

Bislang muss der Heizbeutel durch einen Spalt auf der Frontseite der Heizung eingedrückt werden, wobei der Beutel zusammengedrückt werden muss. Nach dem Einlegen wird das Schlauchssystem befüllt, so dass sich der Heizbeutel in der Heizung ausdehnt und dadurch an den Innenwandungen der Heizung anliegt. Der Einlegevorgang ist jedoch als solcher sehr aufwendig bedingt durch das umständliche Zusammendrücken und das danach vorzunehmende Befüllen. Auch beim Abrüsten stellt sich für das Bedienpersonal die relativ gesehen zeitaufwendige Aufgabe, zunächst die im Heizbeutel befindliche Flüssigkeit zu verdrängen, um dann den Heizbeutel aus der Heizung durch den Spalt entnehmen zu können. Im befüllten Zustand kann der Heizbeutel bei solchen Heizungen nicht entnommen werden.

Es ist daher die Aufgabe der vorliegenden Erfindung, eine Heizung der eingangs genannten Art in vorteilhafter Weise weiterzubilden, insbesondere dahingehend, dass das Einlegen und Entnehmen eines flüssigkeitsführenden Bauteils in eine Heizung für ein medizinisches Gerät vereinfacht wird.

Diese Aufgabe wird erfindungsgemäß durch eine Heizung mit den Merkmalen des Anspruchs 1 gelöst. Danach ist vorgesehen, dass eine Heizung für ein medizinisches Gerät mit einer Aufnahme für wenigstens einen Teil eines flüssigkeitsführenden Bauteils ausgeführt ist, wobei die Aufnahme den einen Teil eines flüssigkeitsführenden Bauteils zumindest teilweise und/oder im Wesentlichen umgreift und eine Öffnung nach einer Seite hin aufweist, wobei der Teil eines flüssigkeitsführenden Bauteils durch die Öffnung in die Aufnahme eingeführt wird. Das Merkmal der Öffnung nach einer Seite hin kann dabei dahingehend derart ausgebildet sein, dass an einer Seite, die nicht die Frontseite ist, die Hauptöffnung zur Einführung für den Teil des flüssigkeitsführenden Bauteils ist. Es ist dabei zunächst unerheblich, welche Ausrichtung die Öffnung hat, also ob die Öffnung nach unten, zur Seite bzw. links oder rechts oder nach oben weisend ist. Entscheidend ist, dass das Teil durch die Öffnung im Wesentlichen ohne vorheriges Zusammendrücken eingeführt oder eingezogen werden kann und/oder dass beim Entnehmen bzw. Herausziehen das Teil des flüssigkeitsführenden Bauteils nicht entleert werden muss. Das flüssigkeitsführende Bauteil kann vorteilhafterweise als Schlauchset eines als Dialysemaschine ausgeführten medizinischen Gerätes ausgebildet sein.

Besonders vorteilhaft ist es, wenn die Öffnung kopfseitig der Heizung angeordnet ist. Dadurch ergibt sich der Vorteil, dass das Teil des flüssigkeitsführenden Bauteils, also beispielsweise ein aufzuheizender Teilbereich eines Schlauchsets z.B. für das Dialysat und/oder das Substituat von oben nach unten in die Heizung eingezogen werden kann. Dabei ist es für diese vorteilhafte Ausgestaltung ausreichend, wenn die kopfseitige Öffnung im oberen Teil z.B. einer Heizung angeordnet ist, wobei die Heizung ihrerseits nicht zwingend genau senkrecht angeordnet sein muss. Eine schräge Anordnung mit einer Abweichung von z.B. ca. 45° zur Senkrechten, aber ggf. auch mehr, kann ebenfalls vorgesehen sein, wenn dadurch beispielsweise dem Verlauf des Schlauchsets besser entsprochen werden kann. Auch bei einer derartigen schrägen Anordnung der Heizung wird durch die kopfseitige Öffnung vorteilhafterweise erreicht, dass der Teil des in die Heizung einzubringenden Teils des flüssigkeitsführenden Bauteils im wesentlichen von oben nach unten in die Heizung eingezogen wird und bereits durch die Schwerkraft am Herausfallen gehindert wird. Es kann jedoch in besonders vorteilhafter Ausgestaltung vorgesehen sein, das die Heizung senkrecht z.B. an einer Bedienseite einer Dialysemaschine und dementsprechend die Öffnung an der Oberseite der Heizung den Zugang zu der senkrecht darunter befindlichen Aufnahme für den Teil des flüssigkeitsführenden Bauteils bildet.

Darüber hinaus ist die Öffnung in einer schlitzförmigen Öffnung auf der frontseitigen Seitenfläche der Heizung weitergeführt ist. Dadurch ergibt sich der Vorteil, dass beispielsweise ein bereits angeschlossenes Schlauchset, das einen länglichen Heizabschnitt mit jeweils einem oben und unten befindlichen Schlauchstück aufweist, mit seinem unteren Schlauchstück in der schlitzförmigen Öffnung nach unten gezogen werden kann, wodurch das untere Schlauchstück knickfrei geführt werden kann. Sowohl das untere als auch das obere Schlauchstück eines derartigen Heizabschnitts eines Schlauchsets können somit durch die Ausgestaltung der Heizung berücksichtigt werden, denn das obere Schlauchstück befindet sich nach dem Einlegen im Bereich der Öffnung der Heizung, während das untere Schlauchstück in der schlitzförmigen Öffnung eingeführt ist. Der Heizabschnitt mit seinen Schlauchstücken kann somit ohne weiteres von oben nach unten eingezogen werden, auch wenn die zulaufenden oder ablaufenden Schlauchstücke bzw. das Schlauchset bereits z.B. an eine Dialysemaschine angeschlossen sind.

Besonders vorteilhaft ist es, wenn die schlitzförmige Öffnung auf der frontseitigen Seitenfläche der Heizung sich im Wesentlichen über die gesamte Länge frontseitige Seitenfläche erstreckt. Dadurch kann erreicht werden, dass z.B. das untere Schlauchstück eines Heizabschnittes eines Schlauchsets bis in den unteren Bereich der Heizung heruntergezogen werden kann.

Das flüssigkeitsführende Bauteil ist ein Schlauchset eines Dialysatsystems und/oder eines Substituatsystems ist. Dabei ist das Schlauchset vorteilhafterweise aus Kunststoff und als Einwegteil bzw. Wegwerfteil bzw. Disposable ausgeführt.

Das Teil des flüssigkeitsführenden Bauteils, der in die Heizung eingeführt wird, ist ein Heizbeutel. Ein derartiger Heizbeutel bildet dabei beispielsweise den Teil eines Schlauchsets für die Dialyse, der in die Heizung eingelegt werden soll.

Ferner weist die Aufnahme ein Innenvolumen auf, das dem Volumen des gefüllten Heizbeutels entspricht.

Weiter ist denkbar, dass die Heizung Mittel zur Temperatursteuerung und/oder - regelung und/oder -erfassung aufweist. Hierbei ist insbesondere denkbar, dass wenigstens ein Temperatursensor vorgesehen ist, der sich im Bereich der Aufnahme befindet. Die Mittel zur Temperatursteuerung und/oder -regelung können auch durch die Maschinensteuerung des medizinischen Gerätes gebildet sein, wobei diese z.B. mit dem in der Aufnahme der Heizung angeordneten Temperatursensor in Verbindung stehen.

Des weiteren betrifft die Erfindung ein medizinisches Gerät, insbesondere eine Dialysemaschine mit den Merkmalen des Anspruchs 8. Danach ist vorgesehen, dass ein medizinisches Gerät, insbesondere eine Dialysemaschine, mit wenigstens einer Heizung nach einem der Ansprüche 1 bis 7 versehen ist.

Außerdem betrifft die Erfindung ein Verfahren zum Aufrüsten eines medizinischen Gerätes, insbesondere einer Dialysemaschine, mit den Merkmalen des Anspruchs 9. Dabei wird ein Heizbeutel eines Schlauchsets in eine Heizung nach Anspruch 1 bis 7 eingelegt mit den folgenden und weiteren Schritten:
- Einführen des Heizbeutels durch die Öffnung in die Aufnahme der Heizung;
- Herunterziehen des sich am unteren Ende des Heizbeutels befindlichen Schlauchteils des Schlauchset in der schlitzförmigen Öffnung auf der frontseitigen Seitenfläche der Heizung;
- Nachrutschen des Heizbeutels in die Zielposition in der Aufnahme der Heizung.

Diese Vorgehensweise erlaubt eine einfache und sichere Aufrüstung in Bezug auf das Einlegen der Heizbeutel und zudem ein einfaches Abrüsten, da der Heizbeutel ohne weiteres Entleeren aus der Heizung herausgezogen werden kann.

Darüber hinaus betrifft die vorliegende Erfindung ein System mit den Merkmalen des Anspruchs 10. Danach ist vorgesehen, dass ein System wenigstens eine Heizung nach einem der Ansprüche 1 bis 7 umfasst, wobei ein Teil eines flüssigkeitsführenden Bauteils in die Heizung eingelegt ist. Dabei handelt es sich um eine Heizung eines medizinischen Gerätes nach Anspruch 8 und bei dem Teil des flüssigkeitsführenden Bauteils um einen Heizbeutel eines Schlauchsets.

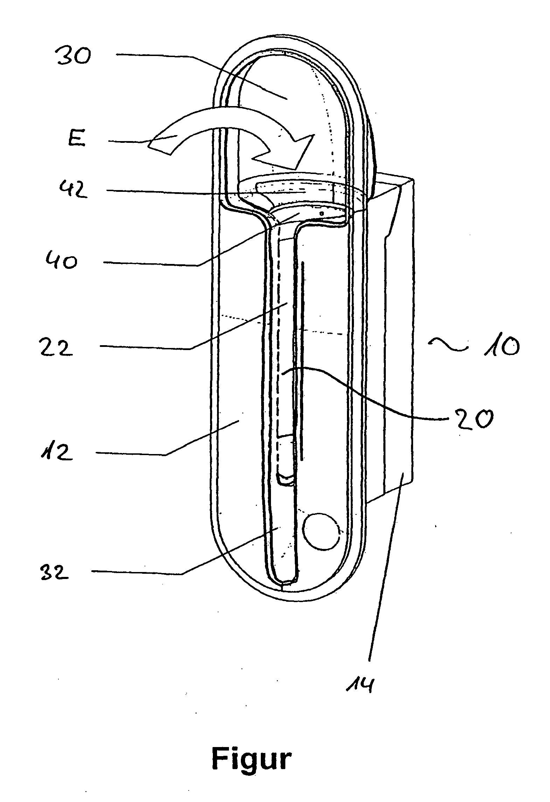

Weitere Einzelheiten und Vorteile sollen nun anhand eines in der Zeichnung dargestellten Ausführungsbeispiels näher erläutert werden.

Die einzige Figur zeigt dabei eine perspektivische Ansicht einer erfindungsgemäßen Heizung für ein medizinisches Gerät.

Dabei ist die erfindungsgemäße Heizung 10 in diesem Ausführungsbeispiel der Erfindung senkrecht an einer nicht näher dargestellten Bedienseite einer Dialysemaschine, beispielsweise einer Akutdialysemaschine angebracht und mit der ebenfalls nicht näher dargestellten Maschinensteuerung der Dialysemaschine verbunden. Die Heizung 10 zeigt somit mit ihrer Frontseite 12 in die gleiche Richtung wie eine Bedienseite der Dialysemaschine.

Die Heizung 10 ist länglich aufgebaut und weist im oberen Bereich eine Öffnung 30 auf, in die ein nicht näher dargestellter Heizbeutel eines Diposable-Schlauchset eingesetzt bzw. eingezogen werden kann, wie dies beim Aufrüsten einer Dialysemaschine notwendig ist. Die Einsetzrichtung E bzw. Einsetzbewegung E ist durch den in der Figur gezeigten Pfeil angedeutet.

Über die kopfseitige Öffnung 30, die in der hier dargestellten Ausführungsform in etwa halbkuppelartig oder domförmig ausgebildet ist, wird der Heizbeutel in die Aufnahme 20 der Heizung 10 eingeführt. Auf der Frontseite 12 weist die Heizung 10 eine schlitzförmige Weiterführung 32 der Öffnung 30 auf, so dass der untere Schlauchteil des Heizbeutels beim Einführen in die Aufnahme 20 durch die schlitzförmige Öffnung 32 auf der Frontseite 12 der Heizung 10 herausgeführt wird. Der untere Schlauchteil des Heizbeutels kann somit bereits konnektiert sein und ist dem Einsetzen des Heizbeutels nicht hinderlich.

Der Heizbeutel eines Schlauchsets wird in die Heizung 10 somit folgendermaßen eingelegt:
Nach dem Konnektieren des Schlauchsets und der obligatorischen Dichtigkeitsprüfung wird der Heizbeutel durch die Öffnung 30 in die Aufnahme 20 der Heizung 10 durch das Bedienpersonal eingelegt, wobei der Zulauf in den Heizbeutel sich unten befindet und in die schlitzförmige Öffnung 32 auf der Frontseite 12 der Heizung 10 eingefädelt wird. Der Zulauf in den Heizbeutel wird gemeinsam mit dem Heizbeutel heruntergezogen, so dass sich der Zulauf in einem Teil der schlitzförmigen Öffnung befindet bzw. dort aus dieser heraustritt, der sich unterhalb des unteren Teils der Aufnahme 20 befindet. Der Heizbeutel rutscht dabei in die Zielposition, also vollständig bis zum unteren Ende in die Aufnahme 20 der Heizung 10 hinein.

Um während des Betriebes die Temperatur im Heizbeutel zu überwachen, ist im oberen Bereich der Aufnahme 20 ein Temperatursensor 40 vorgesehen, der die Temperatur an die Maschinensteuerung der Dialysemaschine weitergibt. Um den Temperatursensor 40 in der Aufnahme 20 zu befestigen, ist ein Elastomerteil 42 vorgesehen, das den Temperatursensor 40 fixiert bzw. aufnimmt. Das Elastomerteil 42 fixiert ferner den nicht näher dargestellten Heizstab, der seinerseits die Heizfläche 22 im Inneren der Aufnahme 20 beheizt. Der Heizbeutel selbst liegt im befüllten Zustand an der Heizfläche 22 an. Dies wird dadurch erreicht, dass die Aufnahme 20 ein Innenvolumen aufweist, das dem Volumen des gefüllten Heizbeutels im wesentlichen entspricht. Somit umgreift die Aufnahme 20 den Heizbeutel im befüllten Zustand nahezu vollständig, ausgenommen nur in den Bereichen der Öffnung 30 und der schlitzförmigen Öffnung 32.

Zum Abrüsten der Maschine, wobei auch das Herausnehmen des Heizbeutels aus der Heizung 10 erfolgt, kann nun einfach der Heizbeutel noch oben aus der Aufnahme 20 durch die Öffnung 30 herausgezogen werden. Ein vorheriges Entleeren ist nicht notwendig. Die gezeigte Ausführungsform der Erfindung ermöglicht es, den Heizbeutel im gefüllten Zustand der Heizung 10 zu entnehmen.

## Patentansprüche

1. Heizung (10), für ein medizinisches Gerät, mit einer Aufnahme (20) für wenigstens einen Teil eines flüssigkeitsführenden Bauteils, wobei die Aufnahme (20) den einen Teil eines flüssigkeitsführenden Bauteils zumindest teilweise und/oder im Wesentlichen umgreift und eine Öffnung (30) nach einer Seite hin aufweist, wobei der Teil eines flüssigkeitsführenden Bauteils durch die Öffnung (30) in die Aufnahme (20) einführbar ist,
wobei die Öffnung (30) in einer schlitzförmigen Öffnung (32) auf der frontseitigen Seitenfläche (12) der Heizung (10) weitergeführt ist, aus der ein am flüssigkeitsführenden Bauteil anschließender Schlauchteil herausführbar ist, **dadurch gekennzeichnet,**
**dass** das Teil des flüssigkeitsführenden Bauteils, das in die Heizung (10) einführbar ist, ein Heizbeutel ist, wobei die Aufnahme (20) ein Innenvolumen aufweist, das dem Volumen des gefüllten Heizbeutels entspricht und sich der Heizbeutel durch Füllen in der Heizung (10) ausdehnt und an dieser anliegt.

2. Heizung (10) für ein medizinisches Gerät nach Anspruch 1, **dadurch gekennzeichnet, dass** die Öffnung (30) kopfseitig der Heizung (10) angeordnet ist.

3. Heizung (10) für ein medizinisches Gerät nach Anspruch 1 oder 2, **dadurch gekennzeichnet, dass** die schlitzförmige Öffnung (32) auf der frontseitigen Seitenfläche (12) der Heizung (10) sich im Wesentlichen über die gesamte Länge der frontseitigen Seitenfläche (12) hin erstreckt.

4. Heizung (10) für ein medizinisches Gerät nach einem der vorhergehenden Ansprüche, **dadurch gekennzeichnet, dass** das flüssigkeitsführende Bauteil ein Teil eines Schlauchsets eines Dialysatsystems und/oder eines Substituatsystems ist.

5. Heizung (10) für ein medizinisches Gerät nach einem der vorhergehenden Ansprüche, **dadurch gekennzeichnet, dass** die Heizung (10) Mittel zur Temperatursteuerung und/oder -regelung und/oder -erfassung (40) aufweist.

6. Heizung nach einem der vorhergehenden Ansprüche, **dadurch gekennzeichnet, dass** die Mittel zur Temperatursteuerung und/oder -regelung und/oder - erfassung (40) mittels eines Elastomerteils (42) am oberen Teil der Aufnahme (20) befestigbar sind.

7. Heizung nach einem der vorhergehenden Ansprüche, **dadurch gekennzeichnet, dass** die Aufnahme (20) eine Heizfläche (22) aufweist, die mittels eines Heizstabes beheizbar ist, wobei der Heizstab mittels des Elastomerteils (42) in der Innenfläche der Aufnahme (20) befestigbar ist.

8. Medizinisches Gerät, insbesondere Dialysemaschine, mit wenigstens einer Heizung (10) nach einem der Ansprüche 1 bis 7.

9. Verfahren zum Aufrüsten eines medizinischen Gerätes, insbesondere einer Dialysemaschine, wobei ein Heizbeutel eines Schlauchsets in eine Heizung (10) nach einem der Ansprüche 1 bis 7 eingelegt wird, mit den folgenden Schritten:
Einführen des Heizbeutels durch die Öffnung (30) in die Aufnahme (20) der Heizung (10);
Herunterziehen des sich am unteren Ende des Heizbeutels befindlichen Schlauchteils des Schlauchset in der schlitzförmigen Öffnung (32) auf der frontseitigen Seitenfläche (12) der Heizung (10) angeordneten schlitzförmigen Öffnung (32);
Nachrutschen des Heizbeutels in die Zielposition in der Aufnahme (20) der Heizung (10);
Befüllen des Heizbeutels, so dass sich der Heizbeutel in der Heizung ausdehnt und dadurch an der Heizung (10) anliegt; und
Herausnehmen des Heizbeutels aus der Heizung (10) durch die Öffnung (30).

10. System umfassend wenigstens eine Heizung (10) nach einem der Ansprüche 1 bis 7, wobei ein Teil eines flüssigkeitsführenden Bauteils in die Heizung (10) einlegbar ist.

## Claims

1. A heating (10), for a medical device, having a receptacle (20) for at least a part of a liquid conducting component, with the receptacle (20) engaging at least partially and/or substantially around the one part of a liquid conducting component and having an opening (30) toward one side, with the part of a liquid conducting component being introduced into the receptacle (20) through the opening (30),
wherein the opening (30) is continued in a slit-shaped opening (32) on the front side surface (12) of the heating (10) from which a hose part can be led out which adjoins the liquid conducting component, **characterized in that** the part of the liquid conducting component which can be introduced into the heating (10) is a heating bag, with the receptacle (20) having an inner volume which corresponds to the volume of the filled heating bag and with the heating bag expanding in the heating (10) by filling and contacting it.

2. A heating (10) for a medical device in accordance with claim 1, **characterized in that** the opening (30) is arranged at the head side of the heating (10).

3. A heating (10) for a medical device in accordance with claim 1 or claim 2, **characterized in that** the slot-shaped opening (32) extends at the front-side side surface (12) of the heating (10) substantially over the total length of the front-side side surface (12).

4. A heating (10) for a medical device in accordance with one of the preceding claims, **characterized in that** the liquid conducting component is a part of a hose kit of a dialysate system and/or of a substituate system.

5. A heating (10) for a medical device in accordance with one of the preceding claims, **characterized in that** the heating (10) has means for the temperature control and/or regulation and/or detection (40).

6. A heating in accordance with one of the preceding claims, **characterized in that** the means for the temperature control and/or regulation and/or detection (40) can be fastened to the upper part of the receptacle (20) by means of an elastomer part (42).

7. A heating in accordance with one of the preceding claims, **characterized in that** the receptacle (20) has a heating surface (22) which can be heated by a heating bar, wherein the heating bar can be fastened in the inner surface of the receptacle (20) by means of the elastomer part (42).

8. A medical device, in particular a dialysis machine, having at least one heating (10) in accordance with one of the claims 1 to 7.

9. A method for the upgrading of a medical device, in particular of a dialysis machine, wherein a heating bag of a hose kit is inserted into a heating (10) in accordance with one of the claims 1 to 7, having the following steps:
introducing the heating bag through the opening (30) into the receptacle (20) of the heating (10);
pulling down the hose part of the hose set located at the lower end of the heating bag in the slot-shaped opening (32) arranged at the front-side side face (12) of the heating (10);
sliding of the heating bag into the target position in the receptacle (20) of the heating (10);
filling the heating bag so that the heating bag expands in the heating and thereby contacts the heating (10); and
removing the heating bag from the heating (10) through the opening (30).

10. A system comprising at least one heating (10) in accordance with one of the claims 1 to 7, wherein a part of a liquid conducting component can be introduced into the heating (10).

## Revendications

1. Chauffage (10), pour un appareil médical, avec un support (20) pour au moins une partie d'un composant conducteur de fluide, le support (20) enveloppant l'une des parties d'un composant conducteur de fluide au moins en partie et/ou principalement et présentant un orifice (30) vers un côté, la partie d'un composant conducteur de fluide étant introductible à travers l'orifice (30) dans le support (20),
l'orifice (30) étant conduit également dans un orifice en forme de fente (32) sur la surface latérale frontale (12) du chauffage (10), à partir de laquelle une partie de tuyau raccordée au composant conducteur de liquide est conductible au-dehors, **caractérisé en ce que**
**en ce que** la partie du composant conducteur de fluide qui est introductible dans le chauffage (10) est un sac chauffant, le support (20) présentant un volume intérieur qui correspond au volume du sac chauffant chargé et **en ce que** le sac chauffant s'étend par chargement dans le chauffage (10) et adhère à celui-ci.

2. Chauffage (10) pour un appareil médical selon la revendication 1, **caractérisé en ce que** l'orifice (30) est positionné côté tête du chauffage (10).

3. Chauffage (10) pour un appareil médical selon la revendication 1 ou 2, **caractérisé en ce que** l'orifice en forme de fente (32) s'étend sur la surface latérale frontale (12) du chauffage (10), et ce, principalement sur la longueur complète de la surface latérale frontale (12).

4. Chauffage (10) pour un appareil médical selon une quelconque des revendications précédentes, **caractérisé en ce que** le composant conducteur de liquide est une partie d'un jeu de tuyaux d'un système de dialysat et/ou d'un système de substituant.

5. Chauffage (10) pour un appareil médical selon une quelconque des revendications précédentes, **caractérisé en ce que** le chauffage (10) présente des moyens en vue de la commande et/ou la régulation et/ou la saisie de température (40).

6. Chauffage selon une quelconque des revendications précédentes, **caractérisé en ce que** les moyens en vue de la commande et/ou la régulation et/ou la saisie de température (40) sont fixables à l'aide d'une pièce en élastomère (42) sur la partie supérieure du support (20).

7. Chauffage selon une quelconque des revendications précédentes, **caractérisé en ce que** le support (20) présente une surface chauffante (22) qui est chauffable à l'aide d'une cartouche chauffante, la cartouche chauffante étant fixable à l'aide de la pièce en élastomère (42) sur la surface intérieure du support (20).

8. Appareil médical, notamment machine de dialyse, avec au moins un chauffage (10) selon une quelconque des revendications 1 à 7.

9. Procédé en vue du rééquipement d'un appareil médical, notamment une machine de dialyse, un sac chauffant d'un jeu de tuyaux étant inséré dans un chauffage (10) selon une quelconque des revendications 1 à 7 avec les étapes suivantes :
Introduction du sac chauffant à travers l'orifice (30) dans le support (20) du chauffage (10) ;
Retrait de la partie de tuyau du jeu de tuyaux se trouvant à l'extrémité inférieure du sac chauffant dans l'orifice en forme de fente (32) positionné sur la surface latérale frontale (12) du chauffage (10) ;
Descente ultérieure du sac chauffant dans la position de destination dans le support (20) du chauffage (10) ;
Chargement du sac chauffant de sorte que le sac chauffant s'étire dans le chauffage et adhère ainsi au chauffage ; et
Extraction du sac chauffant hors du chauffage (10) à travers l'orifice (30).

10. Système comprenant au moins un chauffage (10) selon une quelconque des revendications 1 à 7, une partie d'un composant conducteur de liquide étant introductible dans le chauffage (10).
